# FASCICULE DE BREVET EUROPEEN

(11) **EP 1 703 942 B1**
(45) Date de publication et mention de la délivrance du brevet: **27.02.2008**
(21) Numéro de dépôt: 05702578.5
(22) Date de dépôt: 05.01.2005
(51) Int. Cl.: A61N 1/08, H01R 13/453

(54) **SYSTEME DE SECURITE POUR DISPOSITIF D ELECTROSTIMULATION**
SICHERHEITSSYSTEM FÜR ELEKTROSTIMULATIONSGERÄT
SAFETY SYSTEM FOR ELECTROSTIMULATION DEVICE

(30) Priorité: 12.01.2004 CH 402004
(43) Date de publication de la demande: 27.09.2006
(73) Titulaire: COMPEX MEDICAL S.A, 1024 Ecublens (CH)
(72) Inventeur: MULLER, Pierre-Yves, CH-1245 Collonge-Bellerive (CH); SCHÖNENBERGER, Klaus, CH-1024 Ecublens (CH)
(74) Mandataire: Grosfillier, Philippe
(86) Numéro de dépôt international: PCT/IB2005/050048
(87) Numéro de publication internationale: WO 2005/068015

(56) Documents cités:
- WO-A-20/05025012
- FR-A- 1 503 915
- US-A- 3 902 502
- US-A- 4 431 001
- US-A- 5 758 414
- US-A- 5 885 109

## Description

### Domaine de l'invention

La présente invention se rapporte au domaine des dispositifs d'électrostimulation, notamment d'électrostimulation musculaire.
Plus précisément, elle concerne les dispositifs d'électrostimulation qui comprennent un boîtier pourvu d'au moins une prise chargeur et d'une prise de stimulation.

### Etat de la technique

Plusieurs modèles de boîtiers pour dispositifs d'électrostimulation sont présentés sur le site WEB de la demanderesse (www.compex.info).
En général, un boîtier pour dispositif d'électrostimulation comporte une prise chargeur et plusieurs prises stimulation adaptées pour recevoir respectivement une fiche reliée à un chargeur externe et plusieurs fiches reliées à des électrodes de stimulation.
Le boîtier comprend un accumulateur chargeable en courant continu via la prise chargeur.
L'électrostimulation est réalisée au moyen d'électrodes apposées sur la peau de l'utilisateur.
L'intensité de stimulation est limitée à quelques dizaines de milliampères (en général 120 mA au maximum pour un courant d'impulsion).

Un risque majeur est encouru par l'utilisateur lorsque le boîtier est relié à la fois au réseau par le biais du chargeur et à l'utilisateur par le biais des électrodes. Dans cette configuration, en cas de défaut se produisant dans le chargeur externe (défaut d'isolation, défaut d'un composant etc.) l'utilisateur pourrait se trouver directement relié à la tension du secteur, ce qui représente un danger mortel. En effet, une liaison avec le secteur est susceptible de provoquer une fibrillation cardiaque, une syncope, des brûlures, des douleurs, etc.

Le boîtier décrit dans le brevet américain US 4,431,001 offre une solution à ce problème. Il comporte une prise unique pouvant fonctionner alternativement comme prise chargeur ou prise stimulation.
Cette solution présente cependant plusieurs inconvénients : elle n'est pas adapté pour des boîtiers qui comprennent plusieurs prises de stimulation et en outre, la réalisation de la prise à double fonction est relativement complexe.

### Résumé de l'invention

Un objectif de l'invention vise à augmenter la sécurité des dispositifs d'électrostimulation.
Un autre objectif vise à offrir également un haut niveau de sécurité pour des boîtiers qui comprennent plusieurs prises de stimulation.

Ces objectifs sont atteint avec un boîtier pour dispositif d'électrostimulation qui comprend une prise chargeur et une prise stimulation adaptées pour recevoir respectivement une fiche reliée à un chargeur et une fiche reliée à des électrodes de stimulation. Le boîtier selon l'invention est caractérisé par le fait qu'il comprend en outre un élément mobile de verrouillage adapté pour verrouiller alternativement la prise chargeur ou la prise stimulation.

La présence de l'élément mobile de verrouillage rend disponible soit la prise chargeur, soit la ou les prises de stimulation. La disponibilité simultanée des deux types de prise étant impossible.

Des modes de réalisation particulièrement avantageux sont décrits dans les revendications dépendantes 2 à 14.

Le déplacement de l'élément mobile d'une position à l'autre peut se faire soit manuellement, p.ex. à l'aide d'un élément poussoir que l'utilisateur déplace, soit consécutivement à l'insertion d'une fiche dans la prise qui est bloquée. Dans ce dernier cas, l'élément de verrouillage comporte une surface inclinée qui est rendue apparente dans la prise. Lors de l'insertion de la fiche dans la prise, une force est exercée sur la surface inclinée, ce qui entraîne le déplacement de l'élément de verrouillage.

L'invention sera mieux comprise à la lecture de la description détaillée qui va suivre et à l'examen des figures annexées qui représentent, à titre d'exemple non limitatif un mode de réalisation de l'invention.

### Brève liste des figures

La figure 1 présente un premier mode de réalisation de l'invention avec des prises stimulation disponibles.
La figure 2 présente le boîtier de la figure 1 avec une prise chargeur disponible.
La figure 3 présente de manière schématique un principe de verrouillage selon l'invention avec des prises stimulation disponibles.
La figure 4 présente l'objet de la figure 3 avec une prise chargeur disponible.
La figure 5 présente un deuxième mode de réalisation de l'invention.
La figure 6 présente un troisième mode de réalisation de l'invention avec une prise stimulation disponible.
La figure 7 présente l'objet de la figure 6 avec une prise chargeur disponible.
La figure 8 présente un troisième mode de réalisation de l'invention avec des prises stimulation disponibles.
La figure 9 présente l'objet de la figure 8 avec une prise chargeur disponible.
La figure 10 présente un quatrième mode de réalisation de l'invention avec des prises stimulation disponibles.
La figure 11 présente l'objet de la figure 10 avec une prise chargeur disponible.

### Références numériques utilisées dans les figures

1. Boîtier
2. Prise chargeur
3. Prise de stimulation
4. Elément mobile de verrouillage
5. Elément de verrouillage de la prise chargeur
6. Elément de verrouillage de la prise stimulation
7. Elément poussoir
8. Surface inclinée

### Description détaillée

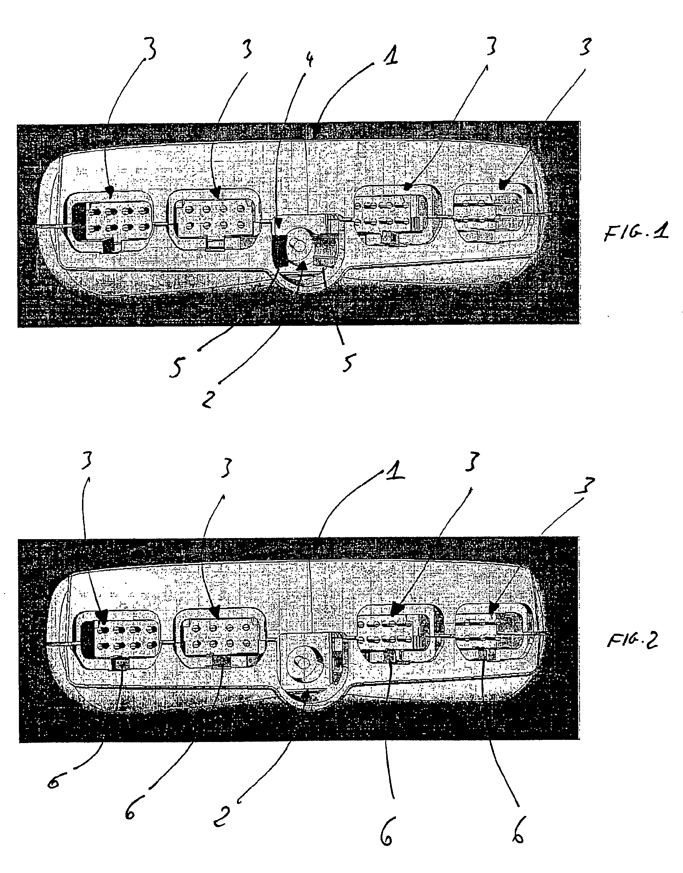

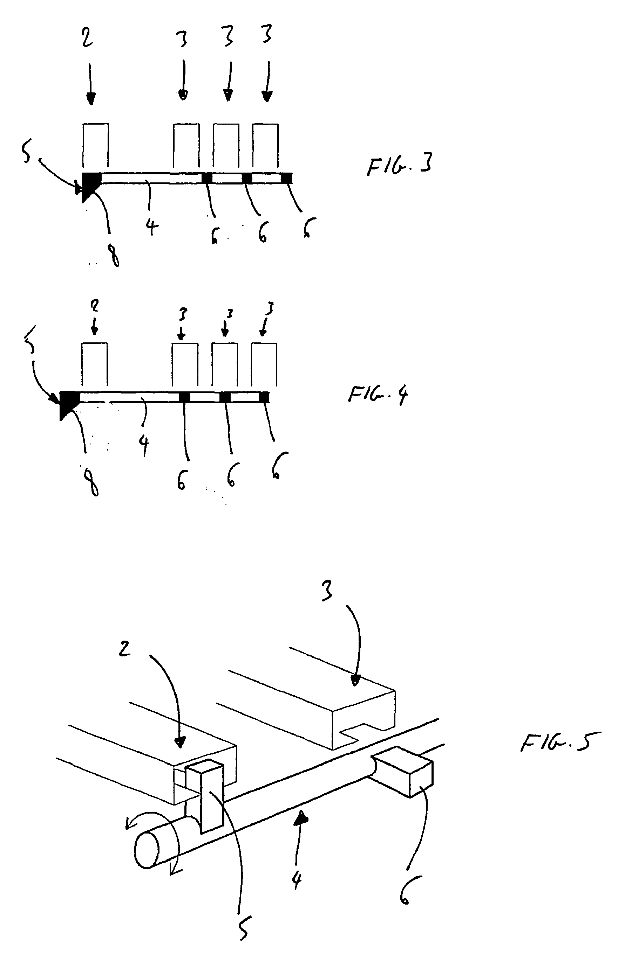

Le boîtier **1** représenté sur les figures 1 et 2 comporte une prise chargeur **2** et quatre prises de stimulation **3.**
Comme on peut le voir aux figures 3 et 4 qui schématisent notamment le concept illustré sur les figures 1 et 2, le boîtier **1** comprend deux éléments de verrouillage **4** en forme de barreau, mobiles en translation selon une direction parallèle à la ligne au qui passe par les prises **2,3.** Chaque barreau **4** comporte une série de dents **6** adaptées pour verrouiller les prises stimulation **6.** L'extrémité de chaque barreau **4** qui débouche dans la prise chargeur **2** comporte une surface inclinée **8.** Lorsque la fiche chargeur est insérée dans la prise correspondante **2,** une force est exercée sur la surface inclinée **8,** ce qui entraîne les barreaux en vers les prises stimulation **3,** verrouillant simultanément leur accès.
De préférence, des ressorts (non illustrés) sont associés aux barreaux de verrouillage **4.** Ils sont disposés de manière à ramener les barreaux de verrouillage **4** lorsque toutes les fiches sont retirées du boîtier **1.**

Les éléments de verrouillage **6** peuvent se présenter sous forme de dents, comme indiqué précédemment. Alternativement, le barreau de verrouillage **4** peut comporter des orifices (non illustrés) au travers desquels les prises peuvent passer. Dans ce cas, les éléments de verrouillage sont constitués par les éléments de barreau qui se situent entre les orifices.

Le mode de réalisation schématiquement illustré à la figure 5 comprend une tige de verrouillage **4** qui pivote autour de son axe principal. Les éléments de verrouillage **6** sont angulairement distants de 90° de sorte que les prises chargeur **2** et stimulation **3** sont alternativement verrouillées.

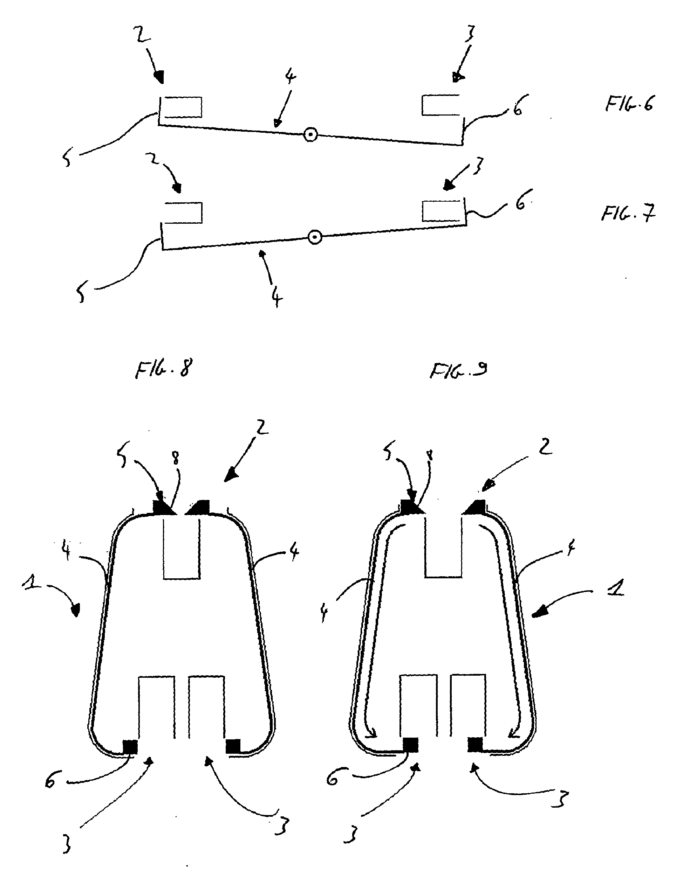

Le mode de réalisation de la figure 6 présente également une tige de verrouillage **4** pivotante, mais selon une direction perpendiculaire à l'axe principal de la tige.
Dans le mode de réalisation des figures 8 et 9, la prise chargeur **2** se situe sur un côté du boîtier **1** opposé à celui où se trouvent les prises stimulation **3.** L'élément de verrouillage **4** est constituée d'une languette semi-rigide, qui se déplace consécutivement à la force exercée sur la surface inclinée **8** par la fiche chargeur.

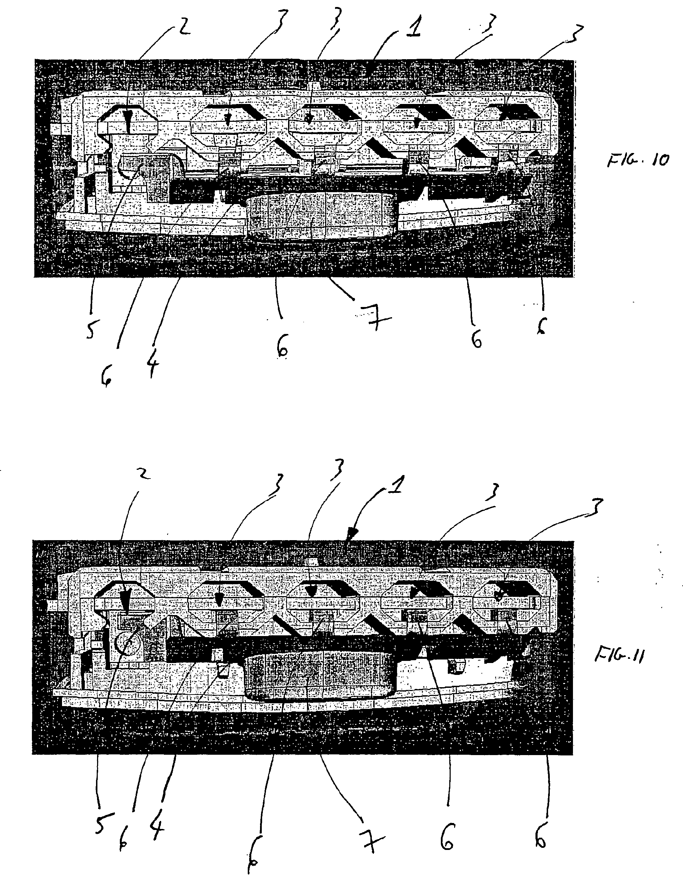

Le mode de réalisation des figures 10 et 11 présente un élément de verrouillage **4** mobile selon une direction verticale. Dans la configuration illustrée à la figure 2, un ressort (non-illustré) maintient l'élément mobile **4** dans une position inférieure (position de repos). Le déplacement vers le haut de l'élément mobile **4** se fait manuellement par l'intermédiaire d'une élément poussoir **7** que l'utilisateur doit pousser vers le haut.

L'élément de verrouillage **5** de la prise chargeur **2** est en forme de L inversé. Quatre éléments de verrouillage **6** de prise stimulation **3** sont disposés le long de l'élément de verrouillage **4,** perpendiculairement à celui-ci.
En position de repos (figure 10), l'élément de verrouillage de la prise chargeur **5** bloque la prise chargeur **2.** Les prises de stimulation **3** sont disponibles et peuvent recevoir des fiches de stimulation.
Si l'utilisateur souhaite insérer la prise de chargeur, il doit préalablement retirer les fiches de stimulation, pousser l'élément poussoir **7** vers le haut et insérer la fiche d'alimentation.
Une fois cette opération effectuée (figure 11), les prises de stimulation sont bloquées par les éléments de verrouillage correspondants **6.**

Selon une variante non-illustrée de l'invention, l'élément poussoir 7 est supprimé. Dans ce cas, l'élément de verrouillage 5 de la prise chargeur 2 comporte une surface inclinée de forme et de fonction identique à celle décrite précédemment.

Il va de soi que l'invention ne se limite pas aux exemples discutés précédemment.

## Revendications

1. Boîtier (1) pour dispositif d'électrostimulation comprenant une prise chargeur (2) et une prise stimulation (3) adaptées pour recevoir respectivement une fiche reliée à un chargeur et une fiche reliée à une électrode de stimulation, **caractérisé par le fait qu'**il comprend un élément mobile de verrouillage (4) adapté pour verrouiller alternativement la prise chargeur (2) ou la prise stimulation (3).

2. Boîtier (1) selon la revendication 1 comprenant plusieurs prises stimulation (3), l'élément de verrouillage (4) étant adapté pour verrouiller alternativement la prise chargeur (2) ou, en une seule fois, les prises de stimulation (3).

3. Boîtier (1) selon la revendication 1 ou 2 **caractérisé par le fait que** l'élément de verrouillage (4) présente une surface inclinée disposée dans une prise de manière à ce que l'insertion d'une fiche dans ladite prise exerce une force sur la surface inclinée de façon à déplacer suffisamment l'élément de verrouillage (4) pour assurer le verrouillage de l'autre prise.

4. Boîtier (1) selon la revendication 3 **caractérisé en ce que** l'élément de verrouillage comprend un barreau muni d'éléments de verrouillage, ledit barreau étant mobile selon une direction parallèle à la ligne qui passe par les prises, chaque élément de verrouillage étant disposé sur le barreau de manière à permettre le verrouillage d'une prise.

5. Boîtier (1) selon la revendication 4 **caractérisé en ce que** ladite surface inclinée est disposée à une extrémité du barreau.

6. Boîtier (1) selon la revendication 5 comprenant deux barreaux disposés dans le prolongement l'un de l'autre et séparés au niveau de la prise chargeur.

7. Boîtier (1) selon la revendication 3 **caractérisé en ce que** la prise chargeur et les prises stimulation sont disposées sur deux côtés opposés du boîtier (1), l'élément de verrouillage étant rendu mobile le long d'un chemin curviligne.

8. Boîtier (1) selon l'une quelconque des revendications 1 à 3 **caractérisé en ce que** l'élément de verrouillage comprend un barreau muni d'éléments de verrouillage, ledit barreau étant monté pivotant.

9. Boîtier (1) selon la revendication 8 **caractérisé en ce que** le barreau est monté pivotant autour de son axe principal, les éléments de verrouillage étant disposés vers les extrémités du barreau.

10. Boîtier (1) selon la revendication 8 **caractérisé en ce que** le barreau est monté pivotant autour d'un axe dirigé perpendiculaire à son axe principal, les éléments de verrouillage étant disposés le long de l'élément de verrouillage.

11. Boîtier (1) selon l'une quelconque des revendications 1 à 3 **caractérisé en ce que** l'élément mobile (4) est adapté pour se déplacer en translation selon une direction perpendiculaire à la ligne selon laquelle les prises (2,3) sont disposées.

12. Boîtier (1) selon la revendication 11 **caractérisé en ce que** l'élément de verrouillage comprend un barreau muni d'éléments de verrouillage.

13. Boîtier (1) selon la revendication 11 ou 12 **caractérisé en ce que** l'élément de verrouillage comprend un élément poussoir qui, lorsqu'il est activé par un utilisateur, permet de libérer une prise.

14. Boîtier (1) selon l'une quelconque des revendications précédentes comprenant des moyens de rappel, par exemple du type ressort, disposés de manière à ramener l'élément de verrouillage (4) dans sa position d'origine une fois la fiche retirée de sa prise correspondante.

## Claims

1. A housing (1) for an electrostimulation device comprising a charger plug (2) and a stimulation plug (3), designed to receive respectively a connector linked to a charger and a connector linked to a stimulation electrode, **characterized in that** it comprises a mobile locking element (4) designed to alternately lock the charger plug (2) or the stimulation plug (3).

2. The housing (1) as claimed in claim 1, comprising a number of stimulation plugs (3), the locking element (4) being designed to alternately lock the charger plug (2) or, in a single action, the stimulation plugs (3).

3. The housing (1) as claimed in claim 1 or 2, **characterized in that** the locking element (4) presents an inclined surface located in a plug so that the insertion of a connector into said plug exerts a force on the inclined surface so as to sufficiently displace the locking element (4) to ensure that the other plug is locked.

4. The housing (1) as claimed in claim 3, **characterized in that** the locking element comprises a strip provided with locking elements, said strip being mobile in a direction parallel to the line passing through the plugs, each locking element being positioned on the strip so as to enable a plug to be locked.

5. The housing (1) as claimed in claim 4, **characterized in that** said inclined surface is located at one end of the strip.

6. The housing (1) as claimed in claim 5, comprising two strips located in the extension of each other and separated at the charger plug.

7. The housing (1) as claimed in claim 3, **characterized in that** the charger plug and the stimulation plugs are located on two opposite sides of the housing (1), the locking element being made mobile along a curvilinear path.

8. The housing (1) as claimed in any one of claims 1 to 3, **characterized in that** the locking element comprises a strip provided with locking elements, said strip being mounted in pivoting fashion.

9. The housing (1) as claimed in claim 8, **characterized in that** the strip is mounted pivoting about its main axis, the locking elements being located towards the ends of the strip.

10. The housing (1) as claimed in claim 8, **characterized in that** the strip is mounted pivoting about an axis directed perpendicular to its main axis, the locking elements being located along the locking element.

11. The housing (1) as claimed in any one of claims 1 to 3, **characterized in that** the mobile element (4) is designed to be displaced in translation in a direction perpendicular to the line on which the plugs (2, 3) are located.

12. The housing (1) as claimed in claim 11, **characterized in that** the locking element comprises a strip provided with locking elements.

13. The housing (1) as claimed in claim 11 or 12, **characterized in that** the locking element comprises a thrust element which, when activated by a user, releases a plug.

14. The housing (1) as claimed in any one of the preceding claims, including return means, for example of the spring type, positioned so as to return the locking element (4) to its original position once the connector has been removed from its corresponding plug.

## Patentansprüche

1. Gehäuse (1) für ein Elektrostimulationsgerät mit einem Ladestecker (2) und einem Stimulationsstecker (3), die dazu ausgelegt sind, einen mit einem Ladegerät verbundenen Verbinder bzw. einen mit einer Stimulationselektrode verbundenen Verbinder aufzunehmen, **dadurch gekennzeichnet, dass** es ein bewegliches Verriegelungselement (4) aufweist, das dazu ausgelegt ist, abwechselnd den Ladestecker (2) oder den Stimulationsstecker (3) zu verriegeln.

2. Gehäuse (1) nach Anspruch 1 mit mehreren Stimulationssteckern (3), wobei das Verriegelungselement (4) dazu ausgelegt ist, abwechselnd den Ladestecker (2) oder auf einmal die Stimulationsstecker (3) zu verriegeln.

3. Gehäuse (1) nach Anspruch 1 oder 2, **dadurch gekennzeichnet, dass** das Verriegelungselement (4) eine geneigte Fläche aufweist, die so in einem Stecker angeordnet ist, dass das Einführen eines Verbinders in den Stecker eine Kraft auf die geneigte Fläche ausübt, derart, dass das Verriegelungselement (4) ausreichend verschoben wird, um die Verriegelung des anderen Steckers zu gewährleisten.

4. Gehäuse (1) nach Anspruch 3, **dadurch gekennzeichnet, dass** das Verriegelungselement einen mit Verriegelungselementen versehenen Stab umfasst, der in einer zu der durch die Stecker gehenden Linie parallelen Richtung beweglich ist, wobei jedes Verriegelungselement so auf dem Stab angeordnet ist, dass die Verriegelung eines Steckers gestattet wird.

5. Gehäuse (1) nach Anspruch 4, **dadurch gekennzeichnet, dass** die geneigte Fläche an einem Ende des Stabs angeordnet ist.

6. Gehäuse (1) nach Anspruch 5 mit zwei Stäben, die in der Verlängerung des einen zum anderen angeordnet und auf der Höhe des Ladesteckers getrennt sind.

7. Gehäuse (1) nach Anspruch 3, **dadurch gekennzeichnet, dass** der Ladestecker und die Stimulationsstecker an zwei gegenüberliegenden Seiten des Gehäuses (1) angeordnet sind, wobei das Verriegelungselement entlang einer gekrümmten Bahn beweglich gemacht ist.

8. Gehäuse (1) nach einem der Ansprüche 1 bis 3, **dadurch gekennzeichnet, dass** das Verriegelungselement einen mit Verriegelungselementen versehenen Stab umfasst, der schwenkend montiert ist.

9. Gehäuse (1) nach Anspruch 8, **dadurch gekennzeichnet, dass** der Stab um seine Hauptachse schwenkend montiert ist, wobei die Verriegelungselemente zu den Enden des Stabs hin angeordnet sind.

10. Gehäuse (1) nach Anspruch 8, **dadurch gekennzeichnet, dass** der Stab um eine Achse schwenkend montiert ist, die senkrecht zu seiner Hauptachse ausgerichtet ist, wobei die Verriegelungselemente entlang dem Verriegelungselement angeordnet sind.

11. Gehäuse (1) nach einem der Ansprüche 1 bis 3, **dadurch gekennzeichnet, dass** das mobile Element (4) dazu ausgelegt ist, sich translatorisch in einer senkrecht zur Linie, in der die Stecker (2, 3) angeordnet sind, verlaufenden Richtung zu verschieben.

12. Gehäuse (1) nach Anspruch 11, **dadurch gekennzeichnet, dass** das Verriegelungselement einen mit Verriegelungselementen versehenen Stab umfasst.

13. Gehäuse (1) nach Anspruch 11 oder 12, **dadurch gekennzeichnet, dass** das Verriegelungselement ein Drückerelement umfasst, das die Freigabe eines Steckers gestattet, wenn es von einem Benutzer betätigt wird.

14. Gehäuse (1) nach einem der vorhergegangenen Ansprüche, mit beispielsweise federartigen Rückholmitteln, die so angeordnet sind, dass sie das Verriegelungselement (4) in seine Ausgangsposition zurückholen, wenn der Verbinder aus seinem entsprechenden Stecker herausgezogen ist.
